# EUROPEAN PATENT APPLICATION

(11) **EP 4 675 273 A1**
(43) Date of publication of application: **07.01.2026**
(21) Application number: 23925423.8
(22) Date of filing: 15.12.2023
(51) Int. Cl.: G01N 30/88, G01N 27/447

(54) **METHOD FOR ANALYZING SIALYL SUGAR CHAIN**

(30) Priority: 01.03.2023 JP 2023031067
(71) Applicant: SHIMADZU CORPORATION, Kyoto-shi, Kyoto 604-8511 (JP)
(72) Inventor: NISHIKAZE, Takashi, Kyoto-shi, Kyoto 604-8511 (JP); WATANABE, Jun, Kyoto-shi, Kyoto 604-8511 (JP); SOGABE, Yuji, Kyoto-shi, Kyoto 604-8511 (JP); KINOSHITA, Mitsuhiro, Higashiosaka-shi, Osaka 577-8502 (JP)
(74) Representative: Müller-Boré & Partner Patentanwälte PartG mbB
(86) International application number: PCT/JP2023/044979
(87) International publication number: WO 2024/180872

(57) **Abstract**

A method for analyzing a sialyl glycan to which sialic acid is linked is provided, the method comprising: a step of preparing a first sample containing a first modified body derived from the sialyl glycan and a second sample containing a second modified body derived from the sialyl glycan; a step of analyzing each of the first sample and the second sample by a chromatography method or an electrophoresis method to obtain first analysis data derived from the first sample and second analysis data derived from the second sample; a step of comparing the first analysis data and the second analysis data; and a step of determining that the sialic acid linked to the sialyl glycan includes at least one selected from the group consisting of α2,3-sialic acid, α2,8-sialic acid, and α2,9-sialic acid, when a mobility attributed to the first modified body shown in the first analysis data and a mobility attributed to the second modified body shown in the second analysis data are different, wherein the first modified body is a compound generated by esterification, amidation, or both of the sialic acid linked to the sialyl glycan, and the second modified body is a compound generated by esterification, amidation, or both of the sialic acid linked to the sialyl glycan, other than the α2,3-sialic acid, the α2,8-sialic acid, and the α2,9-sialic acid.

## Description

### [Technical Field]

The present invention relates to a method for analyzing sialyl glycans.

### [Background Art]

Sialic acid is also contained in glycoproteins in vivo, and in such cases, it mainly exists at the terminal ends of glycan chains. Since sialic acid is typically positioned on the outside of glycoprotein molecules, it is often involved in recognition by other molecules. Sialic acid may have different linkage types with adjacent sugars. For example, in human N-linked glycans, α2,3- and α2,6- linkage types are primarily known. In O-linked glycans and glycosphingolipids, α2,8- and α2,9- linkage types are known in addition to these. Analysis of the linkage types of sialic acid is important because sialic acids can be recognized by different molecules and may have different roles depending on the difference in linkage types.

### [Citation List]

### [Patent Literature]

[Patent Literature 1] JP 2016-194500 A
[Patent Literature 2] JP 2019-152475 A

### [Non-Patent Literature]

[Non-Patent Literature 1] Nishikaze T., Tsumoto H., Sekiya S., Iwamoto S., Miura Y., Tanaka K., Analytical Chemistry, 2017, 89, 2353-2360.

### [Summary of Invention]

### [Technical Problem]

Analyzing sialyl glycans containing sialic acid is not easy because sialic acid has a negative charge and is easily degraded. Furthermore, since the mass (molecular weight) of the glycan does not change depending on the difference in the linkage type of sialic acid, it is not possible to distinguish and analyze the linkage types by mass spectrometry.

Methods for performing linkage-specific modifications to sialic acid have been proposed in order to distinguish and analyze the linkage types of sialic acid. For example, Patent Literature 1 discloses a method for preparing a sample for analysis for analyzing glycans contained in a sample, wherein, when sialic acid is linked to the glycan of an analysis target substance, a first reaction is performed to generate different modified bodies depending on the linkage type of the sialic acid, and in the first reaction, the analysis target substance including the glycan is reacted with an amine having two or more carbon atoms and a dehydration-condensation agent.

Furthermore, Patent Literature 2 discloses a method for preparing a sample including a glycan, comprising: performing a lactonization reaction to lactonize at least a portion of the sialic acid contained in the glycan; and adding, to the sample, an amidation reaction solution including at least one selected from the group consisting of ammonia, amines, and salts thereof, which react with the lactonized sialic acid, and performing an amidation reaction to amidate the lactone of the lactonized sialic acid. Non-Patent Literature 1 discloses a method for preparing a sample used for mass spectrometry, in which a solution containing isopropylamine and a dehydration-condensation agent is added to released N-linked glycans to lactonize α2,3-sialic acid and amidate α2,6-sialic acid.

These methods utilize the property that α2,3-sialic acid is more prone to intramolecular dehydration by a dehydration-condensation agent than α2,6-sialic acid, and molecules with different masses are generated depending on the linkage type of sialic acid. Therefore, the linkage types of sialic acid can be distinguished and analyzed by mass spectrometry.

By the way, in vivo, it is known that differences in the linkage types of sialic acid are involved in various biological phenomena, and for example, it is known that the linkage type of sialic acid changes with canceration. Therefore, discriminating the linkage types of sialic acid as described above is attracting attention for the use of sialic acid as a biomarker, quality control of biopharmaceuticals, and the like, and further improvements (e.g., speeding up, simplification, etc.) in methods for analyzing sialyl glycans are expected.

The present invention has been made in view of the above circumstances, and an object thereof is to provide a method for analyzing sialyl glycans, which is capable of distinguishing the linkage type of sialic acid linked to a sialyl glycan by a chromatography method or an electrophoresis method.

### [Solution to Problem]

As a result of diligent research, the present inventors found that it is possible to distinguish the linkage type of sialic acid linked to a sialyl glycan by analyzing two types of samples, which are modified in different ways depending on the difference in the linkage type of sialic acid, by a chromatography method or an electrophoresis method, thereby completing the present invention.

The present invention relates to:
A method for analyzing a sialyl glycan to which sialic acid is linked, comprising:
a step of preparing a first sample containing a first modified body derived from the sialyl glycan and a second sample containing a second modified body derived from the sialyl glycan;
a step of analyzing each of the first sample and the second sample by a chromatography method or an electrophoresis method to obtain first analysis data derived from the first sample and second analysis data derived from the second sample;
a step of comparing the first analysis data and the second analysis data; and
a step of determining that the sialic acid linked to the sialyl glycan includes at least one selected from the group consisting of α2,3-sialic acid, α2,8-sialic acid, and α2,9-sialic acid, when a mobility attributed to the first modified body shown in the first analysis data and a mobility attributed to the second modified body shown in the second analysis data are different, wherein
the first modified body is a compound generated by esterification, amidation, or both of the sialic acid linked to the sialyl glycan, and
the second modified body is a compound generated by esterification, amidation, or both of the sialic acid linked to the sialyl glycan, other than the α2,3-sialic acid, the α2,8-sialic acid, and the α2,9-sialic acid.

### [Advantageous Effects of Invention]

According to the present invention, it is possible to provide a method for analyzing sialyl glycans, which is capable of distinguishing the linkage type of sialic acid linked to a sialyl glycan.

### [Brief Description of Drawings]

[FIG. 1] FIG. 1 is a flowchart showing an analysis method according to an embodiment of the present invention.
[FIG. 2] FIG. 2 is a fluorescence chromatogram showing the results of HPLC analysis performed in Examples.
[FIG. 3] FIG. 3 is a graph showing the results of analysis by a microchip electrophoresis method performed in Examples.

### [Description of Embodiments]

Hereinafter, an embodiment of the present invention (hereinafter referred to as "the present embodiment") will be described. However, the present embodiment is not limited thereto. In the present specification, the notation "A to Z" means a range including the upper and lower limits (i.e., A or more and Z or less), and when A has no unit description and only Z has a unit description, the unit of A and the unit of Z are the same.

### <<Method for Analyzing Sialyl Glycans>>

A method for analyzing sialyl glycans according to an embodiment of the present invention is
a method for analyzing a sialyl glycan to which sialic acid is linked, comprising:
a step of preparing a first sample containing a first modified body derived from the sialyl glycan and a second sample containing a second modified body derived from the sialyl glycan;
a step of analyzing each of the first sample and the second sample by a chromatography method or an electrophoresis method to obtain first analysis data derived from the first sample and second analysis data derived from the second sample;
a step of comparing the first analysis data and the second analysis data; and
a step of determining that the sialic acid linked to the sialyl glycan includes at least one selected from the group consisting of α2,3-sialic acid, α2,8-sialic acid, and α2,9-sialic acid, when a mobility attributed to the first modified body shown in the first analysis data and a mobility attributed to the second modified body shown in the second analysis data are different, wherein
the first modified body is a compound generated by esterification, amidation, or both of the sialic acid linked to the sialyl glycan, and
the second modified body is a compound generated by esterification, amidation, or both of the sialic acid linked to the sialyl glycan, other than the α2,3-sialic acid, the α2,8-sialic acid, and the α2,9-sialic acid.

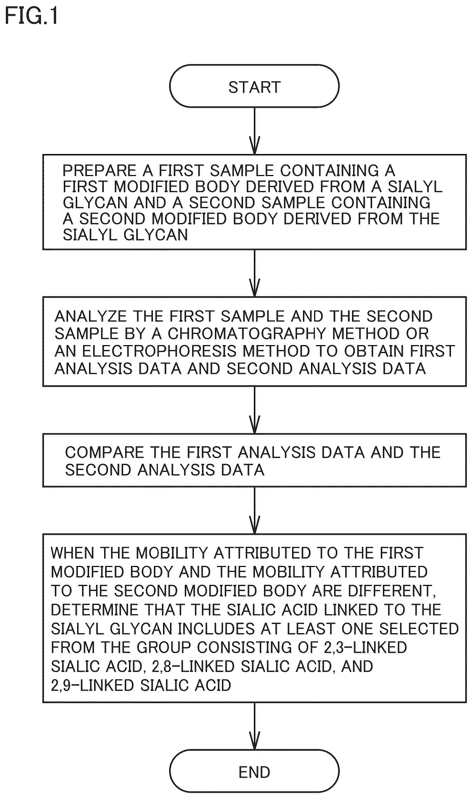

FIG. 1 is a flowchart showing the flow of the analysis method according to one embodiment of the present invention. Hereinafter, each step will be described.

### <Step of Preparing First Sample and Second Sample>

In this step, a first sample containing a first modified body derived from a sialyl glycan and a second sample containing a second modified body derived from the sialyl glycan are prepared.

### (Sialyl Glycan)

In the present embodiment, "sialyl glycan" means a glycan containing at least one sialic acid as a sugar constituting the glycan. The sialyl glycan may be a sialyl glycan constituting a part of a glycoprotein, a sialyl glycan constituting a part of a glycolipid, or a sialyl glycan released therefrom. When the sialyl glycan constitutes a part of a glycoprotein, the sialyl glycan may be an O-linked glycan or an N-linked glycan. In one aspect of the present embodiment, it may be a labeled glycan in which the reducing end of the released sialyl glycan is modified with a labeling compound to enable fluorescence detection or UV detection. Examples of the labeling compound include 2-aminobenzoic acid (2-AA) and 8-aminopyrene-1,3,6-trisulfonic acid (APTS) used in the Examples.

### (First Sample)

In the present embodiment, the first sample may be liquid or solid. The first sample is preferably liquid from the viewpoint of facilitating subsequent steps. In one aspect of the present embodiment, the first sample may be derived from a living body or derived from cells.

### (First Modified Body)

In the present embodiment, the first modified body is derived from the sialyl glycan to be analyzed. The first modified body is a compound generated by esterification, amidation, or both of the sialic acid linked to the sialyl glycan. That is, conceivable sialic acids that are esterified or amidated in the first modified body include α2,3-sialic acid, α2,6-sialic acid, α2,8-sialic acid, and α2,9-sialic acid. In one aspect of the present embodiment, the first modified body can also be understood as a type of glycan. The first modified body may constitute a part of a glycoprotein, constitute a part of a glycolipid, or be a compound released therefrom.

In the present specification, "protein" refers to a general term for molecules in which two or more amino acids are peptide-bonded. In the present specification, protein may include peptides (including oligopeptides and polypeptides) having a small number of amino acid residues, for example, less than 50 amino acid residues, and proteins having a large number of amino acid residues, for example, 50 or more amino acid residues. In the present specification, glycoprotein may include glycopeptide.

Those with a large number of amino acid residues in the peptide chain constituting the glycoprotein may have the peptide chain cleaved by a digestive enzyme or the like. For example, when preparing a sample for liquid chromatography or electrophoresis, the number of amino acid residues in the peptide chain is preferably 30 or less, more preferably 20 or less, and still more preferably 15 or less. The lower limit of the number of amino acid residues in the peptide chain is not particularly limited, but is preferably 2 or more, and more preferably 3 or more.

Examples of the digestive enzyme used when cleaving the peptide chain constituting the glycoprotein include trypsin, Lys-C, arginine endopeptidase, chymotrypsin, pepsin, thermolysin, proteinase K, pronase E, and the like. These digestive enzymes may be used alone as one type or in combination of two or more types. The conditions for cleaving the peptide chain are not particularly limited as long as they do not affect the sialyl glycan to be measured, and an appropriate protocol corresponding to the digestive enzyme to be used is adopted. Before performing the cleavage of the peptide chain, the protein in the sample may be subjected to denaturation treatment or alkylation treatment. The conditions for the denaturation treatment or alkylation treatment are not particularly limited. The peptide chain cleavage treatment may be performed before or after the first reaction or after the second reaction, which will be described later. In one aspect of the present embodiment, the peptide chain may be cleaved by chemical cleavage or the like instead of enzymatic cleavage.

A treatment for blocking amino groups in the glycoprotein in the sample may be appropriately performed. Examples of such a treatment include dimethylamidation and guanidylation of the glycoprotein. This can suppress side reactions such as intramolecular dehydration-condensation that may occur with the amino group or carboxy group at the terminal of the main chain of the protein when the first reaction or the second reaction, which will be described later, is performed.

The first modified body can be generated by esterifying, amidating, or both the sialic acid linked to the sialyl glycan. In one aspect of the present embodiment, the first modified body may be generated by performing linkage-specific modification of sialic acid on the sialyl glycan. Here, "linkage-specific modification of sialic acid" and "linkage-specific sialic acid modification" mean a modification reaction that acts on sialic acid, wherein the chemical structure generated by the modification reaction is different between the case where the sialic acid is α2,3-sialic acid (or α2,8-sialic acid, or α2,9-sialic acid) and the case where it is α2,6-sialic acid. Specifically, the linkage-specific modification of the sialic acid preferably includes a first reaction for lactonizing sialic acid and a second reaction for amidating the lactone structure generated by the first reaction. Hereinafter, the first reaction and the second reaction will be described.

### (First Reaction)

In the first reaction, when sialic acid is linked to a glycoprotein, the sialic acid is selectively lactonized according to the linkage type. In the first reaction, preferably α2,8-sialic acid and α2,9-sialic acid are lactonized in addition to α2,3-sialic acid in the glycan.

The first reaction can be performed by bringing a solution for performing linkage-specific lactonization of sialic acid (hereinafter, also referred to as "lactonization reaction solution") into contact with the sample containing the sialyl glycan. An example of the contact method is to add the lactonization solution to the sample. The lactonization reaction solution preferably includes a dehydration-condensation agent. Simultaneously with the first reaction, it is preferable that α2,6-sialic acid undergoes a modification different from lactonization, preferably amidation or esterification. In this case, the lactonization reaction solution preferably further includes a nucleophile including at least one selected from the group consisting of alcohols, amines, and salts thereof, in addition to the dehydration-condensation agent.

The types and concentrations of the dehydration-condensation agent and the nucleophile should be adjusted so as to selectively cause a dehydration reaction or a nucleophilic reaction based on the linkage type of sialic acid. The lactone generated by intramolecular dehydration of the carboxy group of α2,3-sialic acid is a six-membered ring, and the lactone that can be generated by intramolecular dehydration of the carboxy group of α2,6-sialic acid is a seven-membered ring. α2,3-sialic acid, which produces a six-membered ring that is more stable than a seven-membered ring, is more easily lactonized than α2,6-sialic acid. Furthermore, since the carboxy group of α2,3-sialic acid is located at a position with relatively large steric hindrance compared to the carboxy group of α2,6-sialic acid, large molecules are less likely to react with α2,3-sialic acid compared to α2,6-sialic acid. The types and concentrations of the dehydration-condensation agent and the nucleophile are adjusted so that different modifications are made depending on the linkage type of sialic acid, based on the difference in molecular structure due to the linkage type of sialic acid.

The dehydration-condensation agent preferably includes carbodiimide. This is because when carbodiimide is used, a carboxy group existing at a site with large steric hindrance is less likely to be amidated, compared to the case where a phosphonium-based dehydration-condensation agent (so-called BOP reagent) or a uronium-based dehydration-condensation agent is used as the dehydration-condensation agent. Examples of carbodiimides include N,N'-dicyclohexylcarbodiimide (DCC), N-(3-dimethylaminopropyl)-N'-ethylcarbodiimide (EDC), N,N'-diisopropylcarbodiimide (DIC), 1-tert-butyl-3-ethylcarbodiimide (BEC), N,N'-di-tert-butylcarbodiimide, 1,3-di-p-tolylcarbodiimide, bis(2,6-diisopropylphenyl)carbodiimide, bis(trimethylsilyl)carbodiimide, 1,3-bis(2,2-dimethyl-1,3-dioxolan-4-ylmethyl)carbodiimide (BDDC), and salts thereof (hydrochlorides, etc.).

In order to promote dehydration-condensation by the dehydration-condensation agent and suppress side reactions, it is preferable to use an additive with high nucleophilicity in addition to carbodiimide. In one aspect of the present embodiment, the "additive with high nucleophilicity" can also be understood as a type of dehydration-condensation agent. As the additive with high nucleophilicity, 1-hydroxybenzotriazole (HOBt), 1-hydroxy-7-aza-benzotriazole (HOAt), 4-(dimethylamino)pyridine (DMAP), ethyl 2-cyano-2-(hydroxyimino)acetate (Oxyma), N-hydroxysuccinimide (HOSu), 6-chloro-1-hydroxybenzotriazole (Cl-HoBt), N-hydroxy-3,4-dihydro-4-oxo-1,2,3-benzotriazine (HOOBt), and the like are preferably used.

The amine used as the nucleophile preferably includes a primary and/or secondary alkylamine having two or more carbon atoms. The primary alkylamine is preferably ethylamine, propylamine, isopropylamine, butylamine, sec-butylamine, tert-butylamine, or the like. The secondary alkylamine is preferably dimethylamine, ethylmethylamine, diethylamine, propylmethylamine, isopropylmethylamine, or the like. From the viewpoint of making it difficult for a carboxy group existing at a site with large steric hindrance, such as the carboxy group of α2,3-sialic acid, to be amidated, it is preferable to use an amine having a branched alkyl group, such as isopropylamine. That is, the nucleophile preferably includes an amine compound having a branched alkyl group or a salt thereof. When an amine is used as the nucleophile in the lactonization reaction solution, the carboxy group of some sialic acids, such as α2,6-sialic acid, is amidated based on the linkage type of sialic acid.

The alcohol used as the nucleophile is not particularly limited, and for example, methanol, ethanol, and the like can be used. When an alcohol is used as the nucleophile in the lactonization reaction solution, the carboxy group of some sialic acids, such as α2,6-sialic acid, is esterified based on the linkage type of sialic acid. The nucleophile may include salts of the aforementioned nucleophiles.

The concentration of the dehydration-condensation agent in the lactonization reaction solution is, for example, preferably 1 mM or more and 5 M or less, and more preferably 10 mM or more and 3 M or less. When carbodiimide and an additive with high nucleophilicity (e.g., Oxyma, HOAt, HOBt, etc.) are used in combination, it is preferable that the respective concentrations are within the above ranges. The concentration of the nucleophile in the lactonization reaction solution is, for example, preferably 0.01 M or more and 20 M or less, and more preferably 0.1 M or more and 10 M or less. The reaction temperature of the first reaction may be about -20°C or more and 100°C or less, and is preferably -10°C or more and 50°C or less.

The first reaction can be carried out in either a liquid phase or a solid phase. When performing the reaction in a liquid phase, it is preferable to perform the reaction in a non-aqueous solvent such as dimethyl sulfoxide (DMSO) or dimethylformamide (DMF). Performing the reaction in a non-aqueous solvent tends to suppress side reactions. The concentration of each component in the liquid phase reaction is not particularly limited, and can be appropriately determined according to the type of dehydration-condensation agent, amine, and the like.

When performing the first reaction in a solid phase, the solid-phase carrier is not particularly limited as long as it can immobilize the sialyl glycan (including the sialyl glycan linked to a protein or lipid). In order to immobilize the sialyl glycan, for example, a solid-phase carrier having an epoxy group, a tosy1 group, a carboxy group, an amino group, or the like as a ligand can be used. A glycoprotein including a glycan may be immobilized using a solid-phase carrier having a hydrazide group, an aminooxy group, or the like as a ligand. By performing the reaction in a state where the sialyl glycan is immobilized on the solid-phase carrier, the reaction solution after the reaction can be easily removed, and the sialic acid can be efficiently modified. When magnetic beads are used as the solid-phase carrier, the magnetic beads to which the sialyl glycan is bound can be collected with a magnet, and excess reagents can be removed, or the beads can be washed with a solvent. When resin is used as the solid-phase carrier, the excess reagent may be removed by passing through a filter, and then the resin may be recovered, or the excess reagent in the supernatant may be removed by precipitating the resin by centrifugation. When the first reaction is performed in a liquid phase, the excess reagent may be removed by ultrafiltration.

The product after the first reaction (hereinafter, sometimes referred to as "intermediate") may be subjected to treatments such as purification, desalting, solubilization, concentration, and drying by a known method as necessary to remove the lactonization reaction solution or reduce the concentration of the lactonization reaction solution.

### (Second Reaction)

The second reaction may be performed by bringing the product (intermediate) after the first reaction into contact with a solution for amidation (hereinafter, also referred to as "amidation reaction solution"). Although the α2,3-linkage and the α2,6-linkage of sialic acid can be distinguishably modified by the first reaction, the lactone structure generated from α2,3-sialic acid may be unstable and tends to return to the original carboxylic acid structure by hydrolysis. By specifically amidating the lactone structure by the second reaction, the lactone structure can be stabilized. At the same time, by the second reaction, the sialic acid undergoes modification with a different mass in a linkage-specific manner, whereby α2,3-sialic acid and α2,6-sialic acid can be distinguished. By performing the second reaction, sialic acid can be modified in a linkage-specific manner with higher specificity and more rapidly. An example of the second reaction may be aminolysis. Aminolysis is a reaction based on the interaction between an amino group and a lactone structure. The aminolysis is suitably performed even under anhydrous conditions, and thus is a reaction different from hydrolysis. In the present specification, ring-opening and amidation of a lactone structure by ammonia, amine, or salts thereof, which are possible even under anhydrous conditions, are referred to as "aminolysis". In one aspect of the present embodiment, "stabilization of lactone structure" and "stabilizing a lactone structure" can also be understood as replacing an unstable lactone structure with another stable structure (methylamide group, etc.) by performing aminolysis.

As another example of the second reaction, the lactone structure may be amidated using a strong dehydration-condensation agent. Examples of the dehydration-condensation agent used in the second reaction include phosphonium-based condensation agents and uronium-based condensation agents, and specifically, benzotriazol-1-yl-oxy-tris(dimethylamino)phosphonium hexafluorophosphate (BOP), benzotriazol-1-yl-oxy-tris(pyrrolidino)phosphonium hexafluorophosphate (PyBOP), 2-(1H-benzotriazol-1-yl)-1,1,3,3-tetramethyluronium hexafluorophosphate (HBTU), 2-(7-aza-1H-benzotriazol-1-yl)-1,1,3,3-tetramethyluronium hexafluorophosphate (HATU), (1-[(1-(cyano-2-ethoxy-2-oxoethylideneaminooxy)dimethylaminomorpholino)]uronium hexafluorophosphate (COMU), 2-(1H-benzotriazol-1-yl)-1,1,3,3-tetramethylaminium tetrafluoroborate (TBTU), tetramethylfluoroformamidinium hexafluorophosphate (TFFH), (7-azabenzotriazol-1-yloxy)tripyrrolidinophosphonium hexafluorophosphate (PyAOP), and the like. A lactone cleavage operation may be interposed before the amidation by these strong dehydration-condensation agents.

The amidation reaction solution preferably includes at least one selected from the group consisting of ammonia, amines, and salts thereof. When using an amine, it is preferable to use an amine different from the amine used in the lactonization reaction solution, or to change the mass by modification with a stable isotope or the like. By using amines with different masses in the first reaction and the second reaction, sialic acid can be amidated so as to have different masses depending on the linkage type. A dehydration-condensation agent is not necessary for aminolysis, and the amidation reaction solution does not need to include a dehydration-condensation agent. The amidation reaction solution may include a dehydration-condensation agent, and for example, the amidation reaction solution may be prepared by adding ammonia, an amine, or a salt thereof without removing the lactonization reaction solution added to the sample in the first reaction. In the second reaction, the lactone structure can be stabilized by such a simple operation.

The amine contained in the amidation reaction solution is preferably a primary amine, more preferably a primary amine having a linear hydrocarbon group, and still more preferably a primary amine having a linear alkyl group. The primary amine having a linear alkyl group contained in the amidation reaction solution is preferably a primary amine having 10 or less carbon atoms, more preferably a primary amine having 7 or less carbon atoms, still more preferably methylamine, ethylamine, propylamine, butylamine, or pentylamine, and most preferably methylamine. From the viewpoint of more efficiently amidating the lactone structure, it is preferable that the amine contained in the amidation reaction solution has a linear structure having no branch (hereinafter, "branch" indicates a branch of a hydrocarbon chain) or has a small number of carbon atoms.

When the amine contained in the amidation reaction solution is a primary amine having an unsaturated chain hydrocarbon group, the unsaturated chain hydrocarbon group preferably includes a double bond, and the unsaturated chain hydrocarbon group more preferably includes an allyl group. The amine is preferably allylamine. The amine contained in the amidation reaction solution may be a primary amine including a hydroxy group, and may be ethanolamine. The amine contained in the amidation reaction solution may include various functional groups other than an alkyl group. The amidation reaction solution may include salts of the aforementioned amines. As a result of the amidation reaction, the glycan is modified to include such a functional group, whereby the modified glycan becomes easier to separate not only by mass spectrometry but also by chromatography or the like.

The concentration of ammonia, amines, and salts thereof in the amidation reaction solution is preferably 0.1 M or more, more preferably 0.3 M or more, still more preferably 0.5 M or more, even more preferably 1.0 M or more, and most preferably 3.0 M or more. As a preferred example, the amidation reaction solution includes ammonia or a primary amine, particularly methylamine, and the concentration of the ammonia or primary amine such as methylamine is preferably 0.1 M or more, more preferably 0.3 M or more, still more preferably 0.5 M or more, even more preferably 1.0 M or more, and most preferably 3.0 M or more. The higher the concentration of ammonia, amines, and salts thereof in the amidation reaction solution, the more efficiently the lactone structure can be amidated. The upper limit value of the concentration of ammonia, amines, and salts thereof in the amidation reaction solution is not particularly limited, but may be, for example, 16 M or less.

The solvent of the amidation reaction solution may be an aqueous solvent or an organic solvent, but is preferably a solvent with a low water content from the viewpoint of preventing hydrolysis of the lactone structure and reliably causing rapid amidation. The solvent of the amidation reaction solution is preferably a dehydrated solvent to which a dehydration operation for reducing the water content has been applied, and more preferably an anhydrous solvent. The solvent of the amidation reaction solution preferably includes at least one of methanol and acetonitrile. In another aspect of the present embodiment, the amidation reaction solution may include water, and the solvent of the amidation reaction solution may be water.

The amidation reaction solution is preferably pH 7.7 or more, more preferably pH 8.0 or more, still more preferably pH 8.8 or more, and most preferably pH 10.3 or more. The higher the pH of the amidation reaction solution, the more efficiently the lactone structure can be amidated. The upper limit value of the pH of the amidation reaction solution is not particularly limited, but may be, for example, 14 or less. When amidating a lactone structure using a strong dehydration-condensation agent, the pH is not particularly limited, and the pH may be adjusted according to the dehydration-condensation agent used.

The second reaction can be completed within several seconds to several minutes. The time for bringing the sample into contact with the amidation reaction solution in order to amidate the lactone structure is preferably less than 1 hour, more preferably less than 30 minutes, still more preferably less than 15 minutes, even more preferably less than 5 minutes, and most preferably less than 1 minute. Preferably, the sample may be simply washed with the amidation reaction solution, or the amidation reaction solution may be temporarily passed through the sample held on a carrier or the like. The lower limit value of the time for bringing the sample into contact with the amidation reaction solution in order to amidate the lactone structure is not particularly limited, but may be, for example, 1 second or more. The time from the end of contact between the sample and the lactonization reaction solution to the end of contact between the sample and the amidation reaction solution is preferably less than 1.5 hours, more preferably less than 1 hour, and still more preferably less than 30 minutes. Since the second reaction is completed in a short time, it is possible to prevent the unstable lactone structure from being decomposed and impairing the quantitative performance in the analysis of the glycan. Furthermore, by setting the reaction time of the second reaction short, the sample can be analyzed more efficiently. The lower limit value of the time from the end of contact between the sample and the lactonization reaction solution to the end of contact between the sample and the amidation reaction solution is not particularly limited, but may be, for example, 1 second or more.

The second reaction can be performed in either a liquid phase or a solid phase. The state of the sample when causing the amidation reaction is not particularly limited as long as the sample can be brought into contact with the amidation reaction solution, but it is preferable to bring the amidation reaction solution into contact with the glycoprotein in a state of being bound or adsorbed to a solid-phase carrier. The solid-phase carrier used for the second reaction is not particularly limited as long as it can immobilize the glycoprotein, and examples thereof include solid-phase carriers that can be used for the first reaction.

The sample after the second reaction may be subjected to treatments such as purification, desalting, solubilization, concentration, and drying by a known method as necessary to remove the amidation reaction solution or reduce the concentration of the amidation reaction solution. Removal of excess reagent after the second reaction can be performed by the method described in the section of the first reaction according to the reaction method of solid phase or liquid phase.

Based on the contents of the first reaction and the second reaction described above, the first modified body can also be understood as a compound generated by bringing at least one selected from the group consisting of ammonia, amines, and salts thereof into contact with an intermediate generated by bringing the sialyl glycan into contact with a dehydration-condensation agent and a nucleophile.

### (Second Sample)

In the present embodiment, the second sample may be liquid or solid. The second sample is preferably liquid from the viewpoint of facilitating subsequent steps. In one aspect of the present embodiment, the second sample may be derived from a living body or derived from cells.

### (Second Modified Body)

In the present embodiment, the second modified body is derived from the sialyl glycan to be analyzed. The second modified body is a compound generated by esterification, amidation, or both of the sialic acid linked to the sialyl glycan, other than the α2,3-sialic acid, the α2,8-sialic acid, and the α2,9-sialic acid. In one aspect of the present embodiment, the second modified body can also be understood as a type of glycan. The second modified body may constitute a part of a glycoprotein, constitute a part of a glycolipid, or be a compound released therefrom. Note that the sialyl glycan from which the second modified body is derived and the sialyl glycan from which the first modified body is derived are usually the same. In one aspect of the present embodiment, when the sialyl glycan to be analyzed does not include α2,3-sialic acid, α2,8-sialic acid, and α2,9-sialic acid, the second modified body may be the same compound as the first modified body.

The second modified body can be generated by esterifying, amidating, or both "sialic acid linked to the sialyl glycan" other than α2,3-sialic acid, α2,8-sialic acid, and α2,9-sialic acid. Here, examples of "sialic acid other than α2,3-sialic acid, α2,8-sialic acid, and α2,9-sialic acid" include α2,6-sialic acid and the like. In one aspect of the present embodiment, the second modified body is preferably a compound generated by a method including the first reaction and a third reaction for ring-opening the lactone structure generated by the first reaction. In another aspect of the present embodiment, the second modified body can also be understood as a compound generated by placing an intermediate, generated by bringing the sialyl glycan into contact with a dehydration-condensation agent and a nucleophile, in a basic environment. For the first reaction, the reagents and reaction conditions described above are used. Hereinafter, the third reaction will be described.

### (Third Reaction)

In the third reaction, the lactone structure generated from α2,3-sialic acid may be ring-opened by hydrolysis to return to the original carboxylic acid structure. The third reaction is preferably performed by placing the intermediate generated after the first reaction in a basic environment.

The basic environment is preferably an environment in which at least one selected from the group consisting of a quaternary ammonium cation, a tertiary amine, a secondary amine, a branched primary amine in which two or more carbon atoms are directly bonded to the carbon atom bonded to the amino group, an alkali metal hydroxide, an alkaline earth metal hydroxide, a tetraalkylammonium hydroxide, guanidine, a guanidine derivative, and salts thereof, and an alkaline buffer is present. The alkaline buffer is preferably at least one selected from the group consisting of Tris buffer, Good's buffer, borate buffer, and carbonate buffer. In one aspect of the present embodiment, the basic environment is more preferably an environment in which t-butylamine, trimethylamine, or both are present. By using such a compound with large steric hindrance, aminolysis is suppressed, and the lactone structure can be efficiently returned to the original carboxylic acid structure.

The basic environment is preferably pH 8 or more, more preferably pH 9 or more, still more preferably pH 9.5 or more, and most preferably pH 10 or more. The higher the pH of the basic environment, the more efficiently the lactone structure can be ring-opened. The upper limit value of the pH of the basic environment is not particularly limited, but may be, for example, 14 or less.

The third reaction can be completed within several seconds to several minutes. The reaction time of the third reaction is preferably 1 hour or less, more preferably 30 minutes or less, still more preferably 10 minutes or less, even more preferably 5 minutes or less, and most preferably 3 minutes or less. The lower limit value of the reaction time of the third reaction is not particularly limited, but may be, for example, 1 second or more.

The third reaction can be performed in either a liquid phase or a solid phase. The state of the sample is not particularly limited as long as the sample can be placed in a basic environment, but it is preferable to place the sialyl glycan in a basic environment in a state of being bound or adsorbed to a solid-phase carrier. The solid-phase carrier used for the third reaction is not particularly limited as long as it can immobilize the sialyl glycan, and examples thereof include solid-phase carriers that can be used for the first reaction.

The sample after the third reaction may be subjected to treatments such as purification, desalting, solubilization, concentration, and drying by a known method as necessary to remove the reagent used in the reaction or reduce the concentration of the reagent used in the reaction. Removal of excess reagent after the third reaction can be performed by the method described in the section of the first reaction according to the reaction method of solid phase or liquid phase.

The method for preparing the first sample and the second sample has been described above. Note that when the sialyl glycan is in the state of a glycoprotein linked to a protein, purification of the glycoprotein and liberation of the sialyl glycan from the glycoprotein, which will be described later, may be performed.

### (Purification of Glycoprotein)

By purifying the glycoprotein, the solution used in the first reaction, the second reaction, and/or the third reaction can be removed, and the efficiency of downstream steps can be improved. The purification method and conditions for the glycoprotein can be appropriately selected depending on the type, properties, molecular weight, and the like of the glycoprotein. The purification of the glycoprotein may be performed by a protein precipitation method. As the precipitating agent, salts such as ammonium sulfate; organic solvents such as acetone, acetonitrile, and chloroform; alcohols such as methanol, propanol, and ethanol; acids such as trichloroacetic acid (TCA), hydrochloric acid, and metaphosphoric acid; water-soluble polymers such as polyethylene glycol and dextran, and combinations thereof can be used. In the protein precipitation method, for example, a precipitating agent is added to a sample containing a glycoprotein, and the mixture is allowed to stand at -20°C or more and 30°C or less for an appropriate time. Thereafter, the precipitate formed by centrifuging the sample that has been allowed to stand may be recovered. The purification of the glycoprotein may be performed by immobilizing the glycoprotein on a solid-phase carrier, or may utilize gel filtration chromatography, ionexchange chromatography, hydrophobic chromatography, affinity chromatography, or the like.

### (Liberation of Sialyl Glycan from Glycoprotein)

As a method for liberating the sialyl glycan from the glycoprotein, enzymatic treatment using O-glycosidase, N-glycosidase, endoglycoceramidase, or the like, and chemical liberation methods such as hydrazinolysis and β-elimination can be used. Before the treatment for liberating the glycan, cleavage of the peptide chain of the glycoprotein may be performed. Modification such as labeling of the reducing end of the glycan with 3-aminoquinoline (3AQ), anthranilic acid (2AA), 1-phenyl-3-methyl-5-pyrazolone (PMP), 8-aminopyrene-1,3,6-trisulfonic acid (APTS), or the like may be performed simultaneously with the liberation of the glycan.

When liberating N-linked glycans from the peptide chain of a glycoprotein, enzymatic treatment with Peptide-N-Glycosidase F (PNGase F), Peptide-N-Glycosidase A (PNGase A), Endo-β-N-acetylglucosaminidase (Endo M), or the like is preferably used. When liberating O-linked glycans from the peptide chain of a glycoprotein, a chemical liberation method is preferably used. The hydrazinolysis method may be performed according to a known method, but for example, anhydrous hydrazine or hydrous hydrazine is added to a sample containing a glycoprotein and heated. The β-elimination method may be performed according to a known method, but for example, ammonium carbamate, saturated ammonia, ammonium carbonate, trifluoromethanesulfonic acid anhydride (anhydrous TFMS), sodium hydroxide, dimethylamine, or the like is added to a sample containing a glycoprotein and heated under alkaline conditions. From the viewpoint of suppressing side reactions due to peeling, it is preferable to release O-linked glycans using ammonium salt powder such as ammonium carbamate. A pyrazolone reagent such as PMP may be allowed to coexist during the reaction under alkaline conditions to release the O-linked glycan and simultaneously label it, thereby suppressing side reactions due to peeling. From the viewpoint of preventing the conversion of sialic acid methylated in the second reaction to carboxylic acid, and from the viewpoint of the liberation efficiency of O-linked glycans, the pH of the β-elimination reaction is preferably pH 11.0 or less, and more preferably pH 10.0 or less. From the viewpoint of suppressing decomposition of sialylated glycans and peeling reaction, the pH of the β-elimination reaction is preferably pH 9.5 or less. The pH when performing the β-elimination reaction is usually pH 7.5 or more.

The liberation of the glycan may be performed in a liquid phase or a solid phase. For example, the glycan cleavage treatment described above may be performed on the glycoprotein immobilized on a solid-phase carrier, and the glycan may be recovered. After performing the glycan cleavage treatment on the glycoprotein bound to a solid-phase carrier having a hydrazide group, the glycan may be released and recovered with a weakly acidic solution. By performing the reaction in a state where the sample is immobilized on a solid-phase carrier, removal of the reaction solution or desalting purification becomes easier, and sample preparation can be simplified.

The released glycan may be purified according to a known method, and for example, the protein and the glycan may be separated by liquid-liquid extraction using an organic solvent such as chloroform. The released glycan can also be easily purified by a solid-phase carrier (column). The released glycan can also be recovered by binding it to a solid-phase carrier having a hydrazide group or an aminooxy group. Examples of the solid-phase carrier having a hydrazide group include "BlotGlyco" manufactured by Sumitomo Bakelite Co., Ltd. The glycan may be purified by adsorbing it onto a carrier for hydrophilic interaction chromatography (hereinafter, also referred to as HILIC). The carrier for HILIC preferably includes an amide group. The released glycan may be purified using a carbon column or a reverse-phase carrier such as C18. The labeled glycan may be purified by adsorbing it onto carbon or C18.

### <Step of Obtaining First Analysis Data and Second Analysis Data>

In this step, each of the first sample and the second sample is analyzed by a chromatography method or an electrophoresis method to obtain first analysis data derived from the first sample and second analysis data derived from the second sample. Here, "first analysis data derived from the first sample" means analysis data obtained by analyzing the first sample by a chromatography method or an electrophoresis method. "Second analysis data derived from the second sample" means analysis data obtained by analyzing the second sample by a chromatography method or an electrophoresis method. It goes without saying that the analysis method used to obtain the first analysis data and the analysis method used to obtain the second analysis data are the same.

The chromatography method used in this step is not particularly limited as long as it is a known method, and any method can be used. In one aspect of the present embodiment, the chromatography method is preferably at least one selected from the group consisting of a liquid chromatography method and a supercritical fluid chromatography method, and more preferably a liquid chromatography method. The analysis equipment and analysis conditions used for the chromatography method are not particularly limited, and examples thereof include the analysis equipment and analysis conditions described in the Examples below. The column used for liquid chromatography is not particularly limited, and hydrophobic reverse-phase columns such as C30, C18, C8, and C4, carbon columns, and normal-phase columns for HILIC can be appropriately used.

The analysis data obtained by the chromatography method is not particularly limited, and may be a chromatogram chart or a retention time obtained from the chromatogram.

The electrophoresis method used in this step is not particularly limited as long as it is a known method, and any method can be used. In one aspect of the present embodiment, the electrophoresis method is preferably at least one selected from the group consisting of a capillary electrophoresis method, an SDS-PAGE method, a microchip electrophoresis method, a two-dimensional electrophoresis method, and an isoelectric focusing electrophoresis method, and more preferably at least one selected from the group consisting of a capillary electrophoresis method, an SDS-PAGE method, and a microchip electrophoresis method. The analysis equipment and analysis conditions used for the electrophoresis method are not particularly limited, and known analysis equipment and analysis conditions can be used.

The analysis data obtained by the electrophoresis method is not particularly limited, and may be a chart showing the result of electrophoresis or a mobility obtained from the chart.

### <Step of Comparing First Analysis Data and Second Analysis Data>

In this step, the first analysis data and the second analysis data are compared. The comparison method is not particularly limited, and examples thereof include a method of superimposing both chromatograms and examining the presence or absence of peaks at different positions, and a method of calculating and examining the difference in retention time between the two.

### <Step of Determining that Sialic Acid Linked to Sialyl Glycan Includes Sialic Acid of a Predetermined Linkage type>

In this step, when a mobility attributed to the first modified body shown in the first analysis data and a mobility attributed to the second modified body shown in the second analysis data are different, it is determined that the sialic acid linked to the sialyl glycan includes at least one selected from the group consisting of α2,3-sialic acid, α2,8-sialic acid, and α2,9-sialic acid. Here, the "mobility" described above is a concept including the peak position and retention time of a chromatogram in a chromatography method, and the mobility and migration time in an electrophoresis method.

The method for attributing mobility in the first analysis data and the second analysis data is not particularly limited. For example, when the chemical structure of the first modified body or the second modified body contained in the sample to be analyzed is presumed, and the mobility derived from the presumed chemical structure is a known mobility, the mobility corresponding to the known mobility in each of the first analysis data or the second analysis data can be determined to be the mobility attributed to the first modified body or the second modified body. When the chemical structure of the first modified body or the second modified body contained in the sample to be analyzed cannot be presumed, analysis data derived from a sample containing the sialyl glycan from which the first modified body and the second modified body are derived (corresponding to "third sample" described later) (corresponding to "third analysis data" described later) can be compared with the first analysis data or the second analysis data, and the changed mobility can be determined to be the mobility attributed to the first modified body or the second modified body. Note that, in the comparison of the first analysis data, the second analysis data, and the third analysis data, if no change in mobility is observed (the mobility matches in all three analysis data), it can be determined that the sample to be analyzed does not contain a sialyl glycan.

In the present embodiment, "when a mobility attributed to the first modified body shown in the first analysis data and a mobility attributed to the second modified body shown in the second analysis data are different" includes, for example, the following cases:
(1) When analysis data is obtained by a chromatography method, and the peak position attributed to the first modified body shown in the first analysis data and the peak position attributed to the second modified body shown in the second analysis data are shifted,
(2) When analysis data is obtained by a chromatography method, and the absolute value of the difference between the retention time attributed to the first modified body shown in the first analysis data and the retention time of the peak attributed to the second modified body shown in the second analysis data is greater than 0,
(3) When analysis data is obtained by an electrophoresis method, and the absolute value of the difference between the mobility attributed to the first modified body shown in the first analysis data and the mobility attributed to the second modified body shown in the second analysis data is greater than 0.

When the sialyl glycan to be analyzed includes α2,3-sialic acid, α2,8-sialic acid, or α2,9-sialic acid, the first modified body and the second modified body have different chemical structures, molecular weights, and charge states, and when both are analyzed by a chromatography method or an electrophoresis method, a difference in mobility appears. That is, when the mobility attributed to the first modified body shown in the first analysis data and the mobility attributed to the second modified body shown in the second analysis data are different, it can be determined that the sialic acid linked to the sialyl glycan includes at least one selected from the group consisting of α2,3-sialic acid, α2,8-sialic acid, and α2,9-sialic acid. On the other hand, when the sialyl glycan to be analyzed includes only sialic acid other than α2,3-sialic acid, α2,8-sialic acid, and α2,9-sialic acid (e.g., α2,6-sialic acid), the first modified body and the second modified body have the same chemical structure, molecular weight, and charge state, and when both are analyzed by a chromatography method or an electrophoresis method, no difference in mobility is observed. That is, when the mobility attributed to the first modified body shown in the first analysis data and the mobility attributed to the second modified body shown in the second analysis data are the same, it can be determined that the sialic acid linked to the sialyl glycan is α2,6-sialic acid.

### <Analysis Method Including Third Sample>

The method for analyzing sialyl glycans according to the present embodiment may further comprise:
a step of preparing a third sample containing the sialyl glycan;
a step of analyzing the third sample by a chromatography method or an electrophoresis method to obtain third analysis data derived from the third sample;
a step of comparing the third analysis data with the first analysis data and the second analysis data; and
a step of determining that the sialic acid linked to the sialyl glycan includes at least one selected from the group consisting of α2,3-sialic acid, α2,8-sialic acid, and α2,9-sialic acid, and the sialic acid other than the α2,3-sialic acid, the α2,8-sialic acid, and the α2,9-sialic acid, when the mobility attributed to the first modified body shown in the first analysis data, the mobility attributed to the second modified body shown in the second analysis data, and the mobility attributed to the sialyl glycan shown in the third analysis data are different.

### (Step of Preparing Third Sample)

The third sample according to the present embodiment includes a sialyl glycan. The sialyl glycan is the sialyl glycan from which the first modified body and the second modified body are derived. The third sample may be liquid or solid. The third sample is preferably liquid. In one aspect of the present embodiment, the third sample may be derived from a living body or derived from cells. The third sample may be prepared at the same timing as the "step of preparing first sample and second sample" described above, or may be prepared at a different timing.

In the step of analyzing the third sample by a chromatography method or an electrophoresis method to obtain third analysis data derived from the third sample, analysis is performed by the same method as the analysis method used for the analysis of the first sample and the second sample to obtain third analysis data. The third analysis data may be obtained at the same timing as the "step of obtaining first analysis data and second analysis data" described above, or may be obtained at a different timing. In the step of comparing the third analysis data with the first analysis data and the second analysis data, each analysis data can be compared with each other by the same method as described above.

When the mobility attributed to the first modified body shown in the first analysis data, the mobility attributed to the second modified body shown in the second analysis data, and the mobility attributed to the sialyl glycan shown in the third analysis data are different, it can be determined that the sialic acid linked to the sialyl glycan includes at least one selected from the group consisting of α2,3-sialic acid, α2,8-sialic acid, and α2,9-sialic acid, and sialic acid other than α2,3-sialic acid, α2,8-sialic acid, and α2,9-sialic acid (e.g., α2,6-sialic acid).

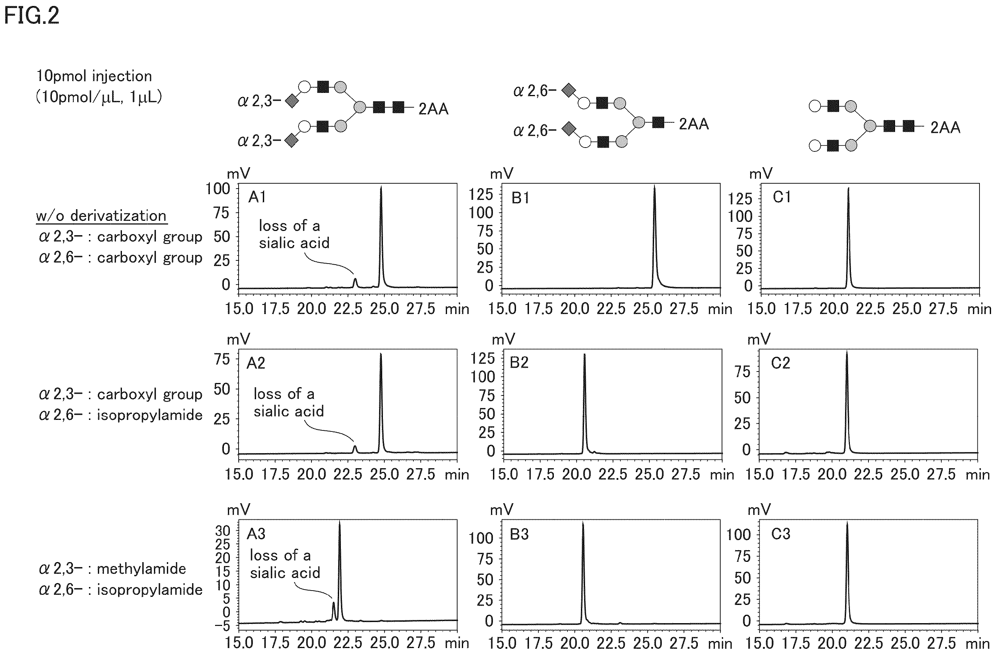

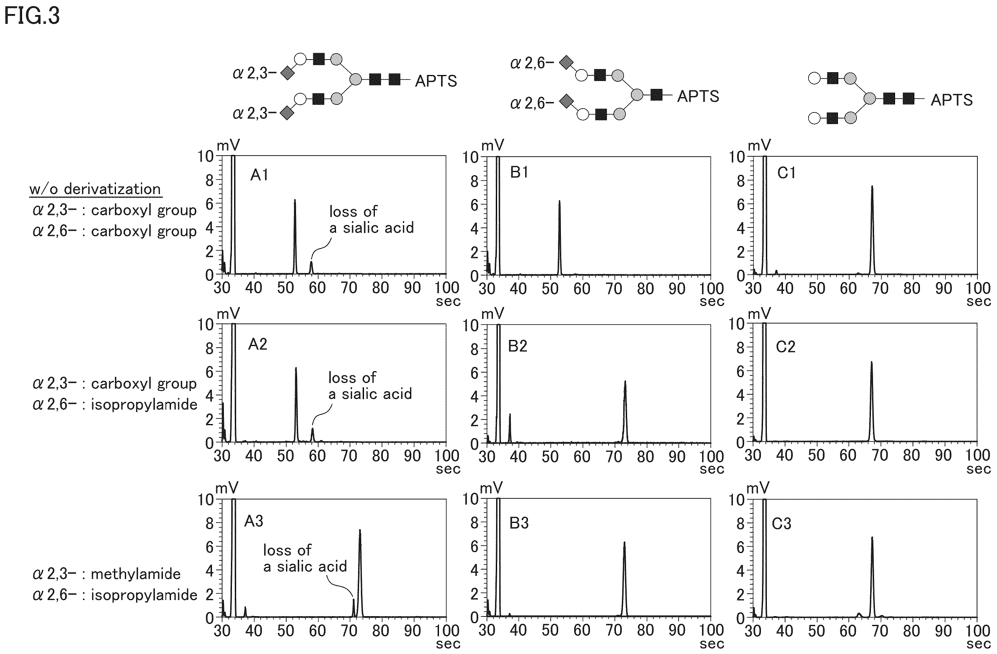

In one aspect of the present embodiment, when the mobility attributed to the first modified body shown in the first analysis data and the mobility attributed to the second modified body shown in the second analysis data are different, and the mobility attributed to the second modified body shown in the second analysis data and the mobility attributed to the sialyl glycan shown in the third analysis data are the same (e.g., A1, A2, A3 in FIGS. 2 and 3), it can be determined that the sialic acid linked to the sialyl glycan includes at least one selected from the group consisting of α2,3-sialic acid, α2,8-sialic acid, and α2,9-sialic acid.

In another aspect of the present embodiment, when the mobility attributed to the first modified body shown in the first analysis data and the mobility attributed to the second modified body shown in the second analysis data are the same, and the mobility attributed to the second modified body shown in the second analysis data and the mobility attributed to the sialyl glycan shown in the third analysis data are different (e.g., B1, B2, B3 in FIGS. 2 and 3), it can be determined that the sialic acid linked to the sialyl glycan includes sialic acid other than α2,3-sialic acid, α2,8-sialic acid, and α2,9-sialic acid (e.g., α2,6-sialic acid).

The method for analyzing sialyl glycans according to the present embodiment has been described above. Conventionally, when analyzing the molecular weight and molecular structure of a measurement target molecule by mass spectrometry, chromatography, or electrophoresis, if the measurement target molecule has a charge, the expected measurement result may not be obtained. For example, when a sialyl glycan containing sialic acid is directly analyzed by capillary electrophoresis (CE) or the like, since the sialic acid is negatively charged, the sialyl glycan is detected earlier than the detection time assumed from the molecular weight of the sialyl glycan, and accurate evaluation of the molecular weight and molecular structure could not be performed. Therefore, it has also been attempted to neutralize (esterify, amidate, etc.) the sialic acid contained in the sialyl glycan to make the charge of the sialic acid neutral before subjecting it to measurement such as capillary electrophoresis. Thus, in conventional analysis methods, although the charge of the measurement target molecule is neutralized before analysis, there was no idea of intentionally returning the once neutralized measurement target molecule to the original "charged state" before subjecting it to analysis. The present inventors reexamined this point and found that sialic acid mainly includes two types, α2,3-sialic acid and α2,6-sialic acid, but in order to distinguish these by CE or microchip electrophoresis, a method of neutralizing both and then returning α2,3-sialic acid to charged sialic acid again is simple, allows for rapid evaluation, and is effective, thereby completing the present invention.

Since sialic acid is located at the glycan terminal, which is easily recognized by other molecules, distinguishing the linkage type of sialic acid can elucidate viral infection or protein-protein interactions on the cell surface. Furthermore, it is also known that the linkage type of sialic acid in glycoproteins changes with canceration, and the use of sialic acid as a biomarker for cancer is also expected. Since the effect of biopharmaceuticals differs depending on glycan modification, distinguishing the linkage type of sialic acid accurately and rapidly can also assist in the quality control of biopharmaceuticals.

### [Examples]

Hereinafter, the present invention will be described in more detail by way of Examples, but the present invention is not limited thereto. The description of % indicates "% by mass" unless otherwise specified.

### [Experiment 1: Method for Analyzing Sialyl Glycans Using HPLC]

### <Reagents>

The following reagents were used.

### (Glycans)

α2,3-Sialyl glycan: α2,3-A2 (manufactured by ACROSCALE Inc.)
α2,6-Sialyl glycan: α2,6-A2GN1 (manufactured by Tokyo Chemical Industry Co., Ltd.)
Neutral glycan: NA2 (manufactured by Sigma-Aldrich)
(Reaction Reagents)
Reagent A: Dimethyl sulfoxide (DMSO) solution of isopropylamine hydrochloride (nucleophile: 15-23%) and 1-hydroxybenzotriazole (additive with high nucleophilicity: 5.0-9.0%)
Reagent B: N-(3-dimethylaminopropyl)-N'-ethylcarbodiimide hydrochloride (>98.0%) (EDC, dehydration-condensation agent)
Reagent C: Aqueous solution of methylamine (16%)

Those included in SialoCapper^{™}-ID Kit (manufactured by Shimadzu Corporation) were used as Reagent A to Reagent C described above.

### <Step of Preparing First Sample and Second Sample>

### (Preparation of First Sample Containing First Modified Body)

Each of the three types of glycans (1 nmol) described above was reacted using SialoCapper^{™}-ID Kit (manufactured by Shimadzu Corporation) according to the attached instruction manual. Specifically, first, a mixture of Reagent A and Reagent B was added to each glycan and reacted with stirring for one hour (First Reaction). At this time, the concentration of the dehydration-condensation agent and the additive with high nucleophilicity in each reaction solution was 500 mM, and the concentration of the nucleophile was 2 M. Next, Reagent C was added to each reaction solution and mixed for several seconds with a vortex mixer (Second Reaction). Thereafter, purification was performed with a HILIC microtip according to the instruction manual, and the solvent was removed with a SpeedVac. As a result of this operation, α2,3-sialic acid (α2,8-sialic acid or α2,9-sialic acid) is methylamidated, and α2,6-sialic acid is isopropylamidated. That is, by this operation, a first modified body derived from each glycan is obtained.

15 mg of 2-aminobenzoic acid (2-AA) was dissolved in 300 µL of 30% acetic acid (AcOH)/DMSO solvent. 12 mg of sodium cyanoborohydride was dissolved using 200 µL of the obtained solution to obtain a reaction solution. 10 µL of the reaction solution was added to each glycan, mixed well, and then reacted at 37 degrees for 18 hours to 2AA-label the reducing end of each glycan. 190 µL of acetonitrile was added to the glycan sample containing the 2AA-labeled glycan, and excess reagent was removed by purification with a HILIC microtip. Specifically, the HILIC microtip was washed with water, and then equilibrated with 90% acetonitrile/0.1% trifluoroacetic acid. The glycan sample diluted with acetonitrile was added to the equilibrated HILIC microtip and passed through by centrifugation. Thereafter, washing was performed with 90% acetonitrile/0.1% trifluoroacetic acid, and finally, the glycan was eluted from the HILIC microtip with water. A first sample containing the first modified body was prepared by the above procedure.

### (Preparation of Second Sample Containing Second Modified Body)

First, a mixture of Reagent A and Reagent B was added to each of the three types of glycans (1 nmol) described above and reacted with stirring for one hour (First Reaction). At this time, the concentration of the dehydration-condensation agent and the additive with high nucleophilicity in each reaction solution was 500 mM, and the concentration of the nucleophile was 2 M. Next, the same amount of water was added instead of Reagent C and mixed for several seconds with a vortex mixer. Thereafter, purification was performed with a HILIC microtip according to the instruction manual. Trimethylamine aqueous solution was added to the eluate from the HILIC microtip so that the final concentration of trimethylamine becomes 14%, mixed for several seconds with a vortex mixer, and the lactone structure was cleaved under a basic environment (pH 12-13) (Third Reaction). Thereafter, the solvent was removed from the reaction solution with a SpeedVac. As a result of this operation, α2,3-sialic acid remains as the original carboxy group, but α2,6-sialic acid is isopropylamidated. That is, by this operation, a second modified body derived from each glycan is obtained.

15 mg of 2-aminobenzoic acid (2-AA) was dissolved in 300 µL of 30% AcOH/DMSO solvent. 12 mg of sodium cyanoborohydride was dissolved using 200 µL of the obtained solution to obtain a reaction solution. 10 µL of the reaction solution was added to each glycan, mixed well, and then reacted at 37 degrees for 18 hours to 2AA-label the reducing end of each glycan. 190 µL of acetonitrile was added to the glycan sample containing the 2AA-labeled glycan, and excess reagent was removed by purification with a HILIC microtip. Specifically, the HILIC microtip was washed with water, and then equilibrated with 90% acetonitrile/0.1% trifluoroacetic acid. The glycan sample diluted with acetonitrile was added to the equilibrated HILIC microtip and passed through by centrifugation. Thereafter, washing was performed with 90% acetonitrile/0.1% trifluoroacetic acid, and finally, the glycan was eluted from the HILIC microtip with water. A second sample containing the second modified body was prepared by the above procedure.

Furthermore, a glycan that underwent only 2AA-labeling without performing the first reaction, the second reaction, and the third reaction described above was prepared as a third sample.

### <Step of Obtaining First Analysis Data and Second Analysis Data>

Each of the obtained first sample and second sample was analyzed by a High-Performance Liquid Chromatography (HPLC) method to obtain first analysis data derived from the first sample and second analysis data derived from the second sample (FIG. 2). Furthermore, the third sample was analyzed by the HPLC method to obtain third analysis data derived from the third sample (FIG. 2). The analysis conditions for HPLC are shown in Table 1 below.

**[Table 1]**

| HPLC Analysis Conditions | |
|---|---|
| System : | Nexera XR (Shimadzu) |
| Column : | GlycanPac AXH-1, Analytical 1.9um, 150mm x 2.1mm (Thermo Fisher Scientific) |
| Mobile Phase: | A) 100 mM Ammonium formate (pH 4.5) |
| | B) Acetonitrile |
| Time Program : B.Conc. | 80% (0-5 min) → 50% (40 min) → 20% (40.01-50 min) →80% (50.01-60 min) |
| Flow Rate : | 0.2 mL/min |
| Column Temp. : | 40 °C |
| Injection Vol. : | 1 µL (ca. glycan 10pmol) |
| Detection : | RF-20A xs (Ex 360 nm, Em 420 nm) |
| Flow Cell : | Semi-micro cell |

### <Step of Comparing First Analysis Data and Second Analysis Data>

FIG. 2 is a fluorescence chromatogram showing the results of the HPLC analysis. In FIG. 2, the horizontal axis indicates retention time (min), and the vertical axis indicates detected fluorescence intensity (mV). At the top of FIG. 2, schematic diagrams of the measurement target molecules are shown. In the schematic diagrams, a diamond indicates sialic acid (here, N-acetylneuraminic acid), a white circle indicates galactose, a black square indicates N-acetylglucosamine, and a gray circle indicates mannose. In FIG. 2, the upper row shows the analysis results of the glycans before modification (third analysis data derived from the third sample). In FIG. 2, the lower row shows the analysis results of the first modified body (first analysis data), and the middle row shows the analysis results of the second modified body (second analysis data). Comparing the first analysis data and the second analysis data, no change in retention time (mobility) was observed for the α2,6-sialyl glycan (B2, B3 in FIG. 2) and the neutral glycan (C2, C3 in FIG. 2). On the other hand, for the α2,3-sialyl glycan (A2, A3 in FIG. 2), a change in retention time (mobility) was observed when the first analysis data and the second analysis data were compared.

### <Step of Determining that Sialic Acid Linked to Sialyl Glycan Includes Sialic Acid of a Predetermined Linkage type>

Hereinafter, the chromatograms in FIG. 2 will be considered. In the case of the A2-type glycan having α2,3-linked sialic acid in the left column, comparing the chromatograms of A2 and A3 in FIG. 2 shows that the retention time is shifted, from which it can be determined that this glycan has α2,3-sialic acid. If it has been confirmed that the target glycan has sialic acid by fractionation using an anion exchange chromatography column such as DEAE (diethylaminoethyl cellulose) in advance, it can also be determined that this glycan has α2,3-sialic acid because the retention time is not shifted by comparing A1 and A2 in FIG. 2. If the presence or absence of sialic acid is unknown, it may not be possible to determine the presence of sialic acid from the comparison of A1 and A2 alone, so it is preferable to make a comprehensive determination by comparing all of A1, A2, and A3. This is because, in the case of a glycan not containing sialic acid, the retention time does not shift depending on the presence or absence of sialic acid modification, as shown in C1, C2, and C3 in the right column of FIG. 2.

In the case of the A2-type glycan having α2,6-sialic acid in the middle column of FIG. 2, comparing the chromatograms of B1 and B2 in FIG. 2 shows that the retention time is shifted, from which it can be determined that this glycan has α2,6-sialic acid. If it has been confirmed that the target glycan has sialic acid by fractionation using an anion exchange chromatography column such as DEAE in advance, it can also be determined that this glycan has α2,6-sialic acid because the retention time is not shifted by comparing B2 and B3. If the presence or absence of sialic acid is unknown, the presence or absence of sialic acid cannot be determined from the comparison of B2 and B3 alone, so in that case, it is preferable to make a comprehensive determination by comparing all of B1, B2, and B3. If the glycan is detected at the same retention time in all of B1, B2, and B3, it can be determined to be a glycan not having sialic acid.

When a single glycan molecule has both α2,3-sialic acid and α2,6-sialic acid, the retention time will be shifted for all of the glycan without sialic acid modification (third sample), the glycan with only α2,6-sialic acid amidated (second modified body, second sample), and the glycan with all sialic acids amidated (first modified body, first sample), so it is preferable to make a determination by comparing all three types of chromatograms.

In addition to α2,3-sialic acid and α2,6-sialic acid, sialic acid linkage types such as α2,8-sialic acid and α2,9-sialic acid also exist depending on the sample, but α2,8-sialic acid and α2,9-sialic acid can also be modified, and their reactivity under sialic acid modification conditions is equivalent to that of α2,3-sialic acid. Therefore, by using this technology, it is possible to determine the presence or absence of sialic acid and to distinguish between α2,6-sialic acid and other sialic acids (α2,3-sialic acid, α2,8-sialic acid, and α2,9-sialic acid).

That is, in the analysis method described above, when the mobility attributed to the first modified body shown in the first analysis data and the mobility attributed to the second modified body shown in the second analysis data are different, it can be determined that the sialic acid linked to the sialyl glycan includes at least one selected from the group consisting of α2,3-sialic acid, α2,8-sialic acid, and α2,9-sialic acid.

Furthermore, in the analysis method described above, when the mobility attributed to the first modified body shown in the first analysis data, the mobility attributed to the second modified body shown in the second analysis data, and the mobility attributed to the sialyl glycan shown in the third analysis data are different, it can be determined that the sialic acid linked to the sialyl glycan includes at least one selected from the group consisting of α2,3-sialic acid, α2,8-sialic acid, and α2,9-sialic acid, and sialic acid other than α2,3-sialic acid, α2,8-sialic acid, and α2,9-sialic acid (e.g., α2,6-sialic acid).

### [Experiment 2: Method for Analyzing Sialyl Glycans Using Microchip Electrophoresis]

### <Reagents>

The same glycans and reaction reagents as in Experiment 1 were used.

### <Step of Preparing First Sample and Second Sample>

### (Preparation of First Sample Containing First Modified Body)

Each of the three types of glycans (1 nmol) described above was reacted using SialoCapper^{™}-ID Kit (manufactured by Shimadzu Corporation) according to the attached instruction manual. Specifically, first, a mixture of Reagent A and Reagent B was added to each glycan and reacted with stirring for one hour (First Reaction). At this time, the concentration of the dehydration-condensation agent and the additive with high nucleophilicity in each reaction solution was 500 mM, and the concentration of the nucleophile was 2 M. Next, Reagent C was added to each reaction solution and mixed for several seconds with a vortex mixer (Second Reaction). Thereafter, purification was performed with a HILIC microtip according to the instruction manual, and the solvent was removed with a SpeedVac. As a result of this operation, α2,3-sialic acid (α2,8-sialic acid or α2,9-sialic acid) is methylamidated, and α2,6-sialic acid is isopropylamidated. That is, by this operation, a first modified body derived from each glycan is obtained.

Each glycan was transferred to a PCR thin-well tube (volume: 0.2 mL), and 50 mM APTS (8-aminopyrene-1,3,6-trisulfonic acid) and 500 mM citric acid in aqueous solution (5 µL) were further added to dissolve each glycan. Next, 1.0 M sodium cyanoborohydride in DMSO solution (5 µL) was added to the tube and reacted at 55°C for 50 minutes to label the reducing end of each glycan with APTS. After the reaction, 140 µL of water was added to the glycan sample containing the labeled glycan to stop the reaction.

The glycan sample diluted with water was transferred to a 1.5 mL tube, 20% tetrabutylammonium bromide/0.5% acetic acid-chloroform solution (200 µL) was added, and vigorously stirred. By this operation, excess labeling reagent dissolves from the aqueous layer into the chloroform layer (liquid-liquid extraction). Next, the tube was centrifuged at 5000 × g for 30 seconds, and the chloroform layer was removed. This liquid-liquid extraction was performed once more, and the recovered aqueous layer was diluted with 300 µL of water and added to HILIC-SPE (HILIC-SPE: Monospin NH2, GL-Science). After centrifuging at 5000 × g for 60 seconds, the carrier was washed with 400 µL of water, and then the labeled glycan was eluted from the carrier with 50 µL of 0.28% ammonium aqueous solution. The labeled glycan was dried in a centrifugal concentrator and stored at -20°C until analysis. A first sample containing the first modified body was prepared by the above procedure.

### (Preparation of Second Sample Containing Second Modified Body)

First, a mixture of Reagent A and Reagent B was added to each of the three types of glycans (1 nmol) described above and reacted with stirring for one hour (First Reaction). At this time, the concentration of the dehydration-condensation agent and the additive with high nucleophilicity in each reaction solution was 500 mM, and the concentration of the nucleophile was 2 M. Next, the same amount of water was added instead of Reagent C and mixed for several seconds with a vortex mixer. Thereafter, purification was performed with a HILIC microtip according to the instruction manual. Trimethylamine aqueous solution was added to the eluate from the HILIC microtip so that the final concentration of trimethylamine becomes 14%, mixed for several seconds with a vortex mixer, and the lactone structure was cleaved under a basic environment (pH 12-13) (Third Reaction). Thereafter, the solvent was removed from the reaction solution with a SpeedVac. As a result of this operation, α2,3-sialic acid remains as the original carboxy group, but α2,6-sialic acid is isopropylamidated. That is, by this operation, a second modified body derived from each glycan is obtained.

Each glycan was transferred to a PCR thin-well tube (volume: 0.2 mL), and 50 mM APTS (8-aminopyrene-1,3,6-trisulfonic acid) and 500 mM citric acid in aqueous solution (5 µL) were further added to dissolve each glycan. Next, 1.0 M sodium cyanoborohydride in DMSO solution (5 µL) was added to the tube and reacted at 55°C for 50 minutes to label the reducing end of each glycan with APTS. After the reaction, 140 µL of water was added to the glycan sample containing the labeled glycan to stop the reaction.

The glycan sample diluted with water was transferred to a 1.5 mL tube, 20% tetrabutylammonium bromide/0.5% acetic acid-chloroform solution (200 µL) was added, and vigorously stirred. By this operation, excess labeling reagent dissolves from the aqueous layer into the chloroform layer (liquid-liquid extraction). Next, the tube was centrifuged at 5000 × g for 30 seconds, and the chloroform layer was removed. This liquid-liquid extraction was performed once more, and the recovered aqueous layer was diluted with 300 µL of water and added to HILIC-SPE (HILIC-SPE: Monospin NH2, GL-Science). After centrifuging at 5000 × g for 60 seconds, the carrier was washed with 400 µL of water, and then the labeled glycan was eluted from the carrier with 50 µL of 0.28% ammonium aqueous solution. The labeled glycan was dried in a centrifugal concentrator and stored at -20°C until analysis. A second sample containing the second modified body was prepared by the above procedure.

Furthermore, a glycan that underwent only APTS-labeling without performing the first reaction, the second reaction, and the third reaction described above was prepared as a third sample.

### <Step of Obtaining First Analysis Data and Second Analysis Data>

First, four reservoirs (R1, R2, R3, and R4) and the separation channel on the microchip were automatically washed with ultrapure water. Next, three reservoirs (R2, R3, and R4; sometimes referred to as "buffer reservoirs") and the separation channel were filled with polymer gel buffer (Gel buffer). Each of the obtained first sample and second sample (3 µL) was electrically injected from reservoir R1 over 45 seconds, and then a separation operation was performed for 140 seconds under the conditions of R1 = 330 V, R2 = 330 V, R3 = 0 V, and R4 = 1485 V. The analysis conditions for the microchip electrophoresis method are shown in Table 2 below. Analysis by the microchip electrophoresis method was performed by the above method to obtain first analysis data derived from the first sample and second analysis data derived from the second sample (FIG. 3). Furthermore, the third sample was analyzed by microchip electrophoresis under the same conditions as above to obtain third analysis data derived from the third sample (FIG. 3).

**[Table 2]**

| Microchip Electrophoresis Analysis Conditions 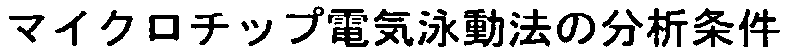 | |
|---|---|
| System : | MCE-202 MultiNA (Shimadzu) |
| Gel buffer : | 50 mM Tris-acetate buffer (pH 8.0) containing 14 % polyethylene glycol (PEG35K, average MW 35000) |
| Fluorescence Detector : | blue LED laser (470nm), long-pass filter (525 nm) |

### <Step of Comparing First Analysis Data and Second Analysis Data>

FIG. 3 is a graph showing the results of the analysis by the microchip electrophoresis method. In FIG. 3, the horizontal axis indicates migration time (sec), and the vertical axis indicates detected fluorescence intensity (unit mV). In FIG. 3, the large peak observed around 30-40 seconds is considered to be a peak derived from residual APTS. At the top of FIG. 3, schematic diagrams of the measurement target molecules are shown. In the schematic diagrams, a diamond indicates sialic acid (here, N-acetylneuraminic acid), a white circle indicates galactose, a black square indicates N-acetylglucosamine, and a gray circle indicates mannose. In FIG. 3, the upper row shows the analysis results of the glycans before modification (third analysis data derived from the third sample). In FIG. 3, the lower row shows the analysis results of the first modified body (first analysis data), and the middle row shows the analysis results of the second modified body (second analysis data). Comparing the first analysis data and the second analysis data, no change in migration time (mobility) was observed for the α2,6-sialyl glycan (B2, B3 in FIG. 3) and the neutral glycan (C2, C3 in FIG. 3). On the other hand, for the α2,3-sialyl glycan (A2, A3 in FIG. 3), a change in migration time (mobility) was observed when the first analysis data and the second analysis data were compared.

### <Step of Determining that Sialic Acid Linked to Sialyl Glycan Includes Sialic Acid of a Predetermined Linkage type>

Hereinafter, the graphs in FIG. 3 will be considered. In the case of the A2-type glycan having α2,3-linked sialic acid in the left column, comparing the graphs of A2 and A3 in FIG. 3 shows that the migration time is shifted, from which it can be determined that this glycan has α2,3-sialic acid. If it has been confirmed that the target glycan has sialic acid by fractionation using an anion exchange chromatography column such as DEAE (diethylaminoethyl cellulose) in advance, it can also be determined that this glycan has α2,3-sialic acid because the migration time is not shifted by comparing A1 and A2 in FIG. 3. If the presence or absence of sialic acid is unknown, it may not be possible to determine the presence of sialic acid from the comparison of A1 and A2 alone, so it is preferable to make a comprehensive determination by comparing all of A1, A2, and A3. This is because, in the case of a glycan not containing sialic acid, the migration time does not shift depending on the presence or absence of sialic acid modification, as shown in C1, C2, and C3 in the right column of FIG. 3.

In the case of the A2-type glycan having α2,6-sialic acid in the middle column of FIG. 3, comparing the graphs of B1 and B2 in FIG. 3 shows that the migration time is shifted, from which it can be determined that this glycan has α2,6-sialic acid. If it has been confirmed that the target glycan has sialic acid by fractionation using an anion exchange chromatography column such as DEAE in advance, it can also be determined that this glycan has α2,6-sialic acid because the migration time is not shifted by comparing B2 and B3. If the presence or absence of sialic acid is unknown, the presence or absence of sialic acid cannot be determined from the comparison of B2 and B3 alone, so in that case, it is preferable to make a comprehensive determination by comparing all of B1, B2, and B3. If the glycan is detected at the same migration time in all of B1, B2, and B3, it can be determined to be a glycan not having sialic acid.

When a single glycan molecule has both α2,3-sialic acid and α2,6-sialic acid, the migration time will be shifted for all of the glycan without sialic acid modification (third sample), the glycan with only α2,6-sialic acid amidated (second modified body, second sample), and the glycan with all sialic acids amidated (first modified body, first sample), so it is preferable to make a determination by comparing all three types of graphs.

In addition to α2,3-sialic acid and α2,6-sialic acid, sialic acid linkage types such as α2,8-sialic acid and α2,9-sialic acid also exist depending on the sample, but α2,8-sialic acid and α2,9-sialic acid can also be modified, and their reactivity under sialic acid modification conditions is equivalent to that of α2,3-sialic acid. Therefore, by using this technology, it is possible to determine the presence or absence of sialic acid and to distinguish between α2,6-sialic acid and other sialic acids (α2,3-sialic acid, α2,8-sialic acid, and α2,9-sialic acid).

That is, in the analysis method described above, when the mobility attributed to the first modified body shown in the first analysis data and the mobility attributed to the second modified body shown in the second analysis data are different, it can be determined that the sialic acid linked to the sialyl glycan includes at least one selected from the group consisting of α2,3-sialic acid, α2,8-sialic acid, and α2,9-sialic acid.

Furthermore, in the analysis method described above, when the mobility attributed to the first modified body shown in the first analysis data, the mobility attributed to the second modified body shown in the second analysis data, and the mobility attributed to the sialyl glycan shown in the third analysis data are different, it can be determined that the sialic acid linked to the sialyl glycan includes at least one selected from the group consisting of α2,3-sialic acid, α2,8-sialic acid, and α2,9-sialic acid, and sialic acid other than α2,3-sialic acid, α2,8-sialic acid, and α2,9-sialic acid (e.g., α2,6-sialic acid).

### [Aspects]

It is understood by those skilled in the art that the plurality of exemplary embodiments and Examples described above are specific examples of the following aspects.

### (Item 1)

A method for analyzing a sialyl glycan to which sialic acid is linked according to one aspect comprises: a step of preparing a first sample containing a first modified body derived from the sialyl glycan and a second sample containing a second modified body derived from the sialyl glycan; a step of analyzing each of the first sample and the second sample by a chromatography method or an electrophoresis method to obtain first analysis data derived from the first sample and second analysis data derived from the second sample; a step of comparing the first analysis data and the second analysis data; and a step of determining that the sialic acid linked to the sialyl glycan includes at least one selected from the group consisting of α2,3-sialic acid, α2,8-sialic acid, and α2,9-sialic acid, when a mobility attributed to the first modified body shown in the first analysis data and a mobility attributed to the second modified body shown in the second analysis data are different, wherein the first modified body is a compound generated by esterification, amidation, or both of the sialic acid linked to the sialyl glycan, and the second modified body is a compound generated by esterification, amidation, or both of the sialic acid linked to the sialyl glycan, other than the α2,3-sialic acid, the α2,8-sialic acid, and the α2,9-sialic acid. According to the method of Item 1, the linkage type of sialic acid linked to a sialyl glycan can be distinguished by a chromatography method or an electrophoresis method.

### (Item 2)

The method according to Item 1, further comprising a step of determining that the sialic acid linked to the sialyl glycan is α2,6-sialic acid, when the mobility attributed to the first modified body shown in the first analysis data and the mobility attributed to the second modified body shown in the second analysis data are the same. According to the method of Item 2, the linkage type of sialic acid linked to a sialyl glycan can be distinguished in more detail by a chromatography method or an electrophoresis method.

### (Item 3)

The method according to Item 1, further comprising: a step of preparing a third sample containing the sialyl glycan; a step of analyzing the third sample by a chromatography method or an electrophoresis method to obtain third analysis data derived from the third sample; a step of comparing the third analysis data with the first analysis data and the second analysis data; and a step of determining that the sialic acid linked to the sialyl glycan includes at least one selected from the group consisting of α2,3-sialic acid, α2,8-sialic acid, and α2,9-sialic acid, and the sialic acid other than the α2,3-sialic acid, the α2,8-sialic acid, and the α2,9-sialic acid, when the mobility attributed to the first modified body shown in the first analysis data, the mobility attributed to the second modified body shown in the second analysis data, and the mobility attributed to the sialyl glycan shown in the third analysis data are different. According to the method of Item 3, the linkage type of sialic acid linked to a sialyl glycan can be distinguished in more detail by a chromatography method or an electrophoresis method.

### (Item 4)

The method according to any one of Items 1 to 3, wherein the first modified body is a compound generated by bringing at least one selected from the group consisting of ammonia, amines, and salts thereof into contact with an intermediate generated by bringing the sialyl glycan into contact with a dehydration-condensation agent and a nucleophile. According to the method of Item 4, the first modified body can be efficiently generated.

### (Item 5)

The method according to Item 4, wherein the dehydration-condensation agent includes carbodiimide. According to the method of Item 5, the first modified body can be generated more efficiently.

### (Item 6)

The method according to Item 4 or 5, wherein the nucleophile includes an amine compound having a branched alkyl group or a salt thereof. According to the method of Item 6, the first modified body can be generated more efficiently.

### (Item 7)

The method according to any one of Items 1 to 6, wherein the second modified body is a compound generated by placing an intermediate, generated by bringing the sialyl glycan into contact with a dehydration-condensation agent and a nucleophile, in a basic environment. According to the method of Item 7, the second modified body can be efficiently generated.

### (Item 8)

The method according to Item 7, wherein the basic environment is an environment in which t-butylamine, trimethylamine, or both are present. According to the method of Item 8, the second modified body can be generated more efficiently.

### (Item 9)

The method according to any one of Items 1 to 8, wherein the chromatography method is a liquid chromatography method. According to the method of Item 9, α-2,3 sialic acid, etc., and α-2,6 sialic acid can be distinguished by the retention time of the chromatogram.

### (Item 10)

The method according to any one of Items 1 to 9, wherein the electrophoresis method is at least one selected from the group consisting of a capillary electrophoresis method, an SDS-PAGE method, and a microchip electrophoresis method. According to the method of Item 10, α-2,3 sialic acid, etc., and α-2,6 sialic acid can be distinguished by the mobility by the electrophoresis method.

## Claims

1. A method for analyzing a sialyl glycan to which sialic acid is linked, comprising:
a step of preparing a first sample containing a first modified body derived from the sialyl glycan and a second sample containing a second modified body derived from the sialyl glycan;
a step of analyzing each of the first sample and the second sample by a chromatography method or an electrophoresis method to obtain first analysis data derived from the first sample and second analysis data derived from the second sample;
a step of comparing the first analysis data and the second analysis data; and
a step of determining that the sialic acid linked to the sialyl glycan includes at least one selected from the group consisting of α2,3-sialic acid, α2,8-sialic acid, and α2,9-sialic acid, when a mobility attributed to the first modified body shown in the first analysis data and a mobility attributed to the second modified body shown in the second analysis data are different, wherein
the first modified body is a compound generated by esterification, amidation, or both of the sialic acid linked to the sialyl glycan, and
the second modified body is a compound generated by esterification, amidation, or both of the sialic acid linked to the sialyl glycan, other than the α2,3-sialic acid, the α2,8-sialic acid, and the α2,9-sialic acid.

2. The method for analyzing a sialyl glycan according to claim 1, further comprising a step of determining that the sialic acid linked to the sialyl glycan is α2,6-sialic acid, when the mobility attributed to the first modified body shown in the first analysis data and the mobility attributed to the second modified body shown in the second analysis data are the same.

3. The method for analyzing a sialyl glycan according to claim 1, further comprising:
a step of preparing a third sample containing the sialyl glycan;
a step of analyzing the third sample by a chromatography method or an electrophoresis method to obtain third analysis data derived from the third sample;
a step of comparing the third analysis data with the first analysis data and the second analysis data; and
a step of determining that the sialic acid linked to the sialyl glycan includes at least one selected from the group consisting of α2,3-sialic acid, α2,8-sialic acid, and α2,9-sialic acid, and the sialic acid other than the α2,3-sialic acid, the α2,8-sialic acid, and the α2,9-sialic acid, when the mobility attributed to the first modified body shown in the first analysis data, the mobility attributed to the second modified body shown in the second analysis data, and the mobility attributed to the sialyl glycan shown in the third analysis data are different.

4. The method for analyzing a sialyl glycan according to any one of claims 1 to 3, wherein the first modified body is a compound generated by bringing at least one selected from the group consisting of ammonia, amines, and salts thereof into contact with an intermediate generated by bringing the sialyl glycan into contact with a dehydration-condensation agent and a nucleophile.

5. The method for analyzing a sialyl glycan according to claim 4, wherein the dehydration-condensation agent includes carbodiimide.

6. The method for analyzing a sialyl glycan according to claim 4, wherein the nucleophile includes an amine compound having a branched alkyl group or a salt thereof.

7. The method for analyzing a sialyl glycan according to any one of claims 1 to 3, wherein the second modified body is a compound generated by placing an intermediate, generated by bringing the sialyl glycan into contact with a dehydration-condensation agent and a nucleophile, in a basic environment.

8. The method for analyzing a sialyl glycan according to claim 7, wherein the basic environment is an environment in which t-butylamine, trimethylamine, or both are present.

9. The method for analyzing a sialyl glycan according to any one of claims 1 to 3, wherein the chromatography method is a liquid chromatography method.

10. The method for analyzing a sialyl glycan according to any one of claims 1 to 3, wherein the electrophoresis method is at least one selected from the group consisting of a capillary electrophoresis method, an SDS-PAGE method, and a microchip electrophoresis method.
